# FASCICULE DE BREVET EUROPEEN

(11) **EP 1 226 438 B1**
(45) Date de publication et mention de la délivrance du brevet: **06.05.2004**
(21) Numéro de dépôt: 00974662.9
(22) Date de dépôt: 06.11.2000
(51) Int. Cl.: G01N 33/543, G01N 33/546

(54) **NANOSPHERES COMPOSITES ET LEURS CONJUGUES AVEC DES BIOMOLECULES**
VERBUNDNANOPARTIKEL SOWIE DEREN KONJUGATE MIT BIOMOLEKÜLEN
COMPOSITE NANOSPHERES AND THEIR CONJUGATES WITH BIOMOLECULES

(30) Priorité: 05.11.1999 FR 9914194
(43) Date de publication de la demande: 31.07.2002
(73) Titulaire: BIO MERIEUX, 69280 Marcy l'Etoile (FR); CENTRE NATIONAL DE LA RECHERCHE SCIENTIFIQUE (CNRS), 75794 Paris Cedex 16 (FR)
(72) Inventeur: ELAISSARI, Abdelhamid, F-69007 Lyon (FR); BOSC, Eric, F-33770 Salles (FR); PICHOT, Christian, F-69960 Corbas (FR); MANDRAND, Bernard, F-69100 Villeurbanne (FR); BIBETTE, Jérôme, F-33000 Bordeaux (FR); MONDAIN-MONVAL, Olivier, F-3300 Bordeaux (FR)
(74) Mandataire: Guerre, Dominique
(86) Numéro de dépôt international: PCT/FR2000/003085
(87) Numéro de publication internationale: WO 2001/033223

(56) Documents cités:
- EP-A- 0 180 384
- EP-A- 0 390 634
- WO-A-99/19000
- US-A- 4 342 739
- US-A- 4 358 388

## Description

La microencapsulation est un procédé utilisé pour l'obtention de petites particules solides enrobées par au moins une couche de polymère. Ce procédé a notamment été utilisé pour l'obtention de poudres inorganiques revêtues d'une couche d'un polymère organique. De tels systèmes sont supposés présenter des propriétés différentes de la somme des propriétés des composants individuels, en particulier de meilleures propriétés mécaniques. Les procédés de microencapsulation ont notamment été utilisés dans le domaine de la préparation de pigments, d'encres, de plastiques et de peintures. Une des applications les plus importantes de particules et pigments encapsulés est trouvée dans le domaine des peintures en émulsion. Mais quand les particules inorganiques obtenues par encapsulation sont magnétisables, cela ouvre des voies particulières dans le domaine de la biologie, par exemple grâce au couplage de protéines ou d'anticorps sur les particules encapsulées pour une utilisation dans des essais de diagnostic. De telles particules sont également utilisées dans des procédés de séparation biochimique. D'une manière générale les particules encapsulées présentent un intérêt comme support, vecteur ou véhicule dans les domaines de l'ingénierie biologique, du diagnostic et de la pharmacie. A cet effet, elles ont été utilisées dans le diagnostic médical comme support solide pour des macromolécules biologiques.

Les particules colloïdales présentent plusieurs avantages par rapport aux supports solides traditionnels, tels que tubes et plaques, notamment parce qu'elles permettent de disposer d'une grande surface pour des interactions spécifiques et parce qu'elles sont facilement modifiables chimiquement pour introduire à leur surface des groupements fonctionnels susceptibles de réagir avec d'autres molécules, par exemple des molécules biologiques telles que des anticorps ou des fragments d'anticorps, des protéines, des polypeptides, des polynucléotides, des acides nucléiques, des fragments d'acides nucléiques, des enzymes ou des molécules chimiques telles que des catalyseurs, des médicaments, des molécules cages, des chélatants.

Parmi les particules colloïdales, les latex magnétiques ont suscité un grand intérêt dans le domaine analytique et sont utilisés par exemple comme moyen pour séparer et/ou détecter des analytes, tels que des antigènes, des anticorps, des molécules biochimiques, des acides nucléiques et autres.

Les particules composites de type polymère/magnétique sont habituellement classées en trois catégories sur un critère de taille: les petites particules ayant un diamètre inférieur à 50 nm, les grosses particules ayant un diamètre supérieur à 2 µm et les particules intermédiaires d'un diamètre compris entre 50 et 1000 nm.

Mais pour qu'elles puissent être considérées comme de bons candidats, en particulier pour une application diagnostique, elles doivent répondre à certains critères. D'un point de vue morphologique, il est préférable qu'elles soient relativement sphériques et que la charge magnétique soit répartie de façon relativement homogène dans la matrice polymère. Elles ne doivent pas s'agréger de manière irréversible sous l'action d'un champ magnétique, ce qui signifie qu'elles puissent être redispersées facilement, rapidement et de manière réversible. De même, elles doivent présenter une densité relativement faible pour réduire le phénomène de sédimentation. Avantageusement, elles doivent présenter une distribution granulométrique étroite. On parle encore de particules monodisperses ou isodisperses.

Ainsi, en raison de leur taille et de leur densité, les grosses particules magnétiques en suspension dans une phase liquide ont tendance à rapidement sédimenter. Par ailleurs, elles tendent à s'agréger après avoir été soumises à un champ magnétique car elles sont susceptibles d'avoir été de ce fait magnétisées de manière permanente. On parle d'aimantation rémanante. Elles ne constituent donc pas un bon candidat.

A contrario, les petites particules magnétiques ont tendance à rester en suspension du fait de leur mouvement Brownien et sont difficilement attirées, voire pas du tout, par un aimant, en particulier si le champ magnétique appliqué est relativement faible. Elles ne sont donc pas bien appropriées pour les utilisations développées ci-dessus.

Il existe donc un intérêt évident à produire des particules composites de type polymère/magnétique, présentant une taille intermédiaire entre 50 et 1000 nm, qui à la fois pallient les inconvénients précités et répondent notamment aux critères établis ci-dessus. Mais l'invention n'est pas limitée à des particules composites magnétisables, comme décrit ci-après.

On peut citer les particules Dynal (nom commercial). Ces particules sont des microsphères constituées d'un coeur poreux de polystyrène et d'oxydes de fer, les oxydes de fer ayant été déposés par imprégnation au niveau des pores disponibles à la surface du polystyrène, et d'une enveloppe en un autre polymère qui encapsule les oxydes de fer des microsphères poreuses. Elles présentent un diamètre respectivement de 2,8 µm (particules M280) et de 4,5 µm (particules M450) et sont relativement uniformes en taille. Elles sont donc considérées comme des particules isodisperses mais en raison de leur taille élevée présentent les inconvénients précités, principalement le phénomène de sédimentation. De plus, leur surface spécifique est faible.

La demande de brevet EP 0 390 634 décrit des microsphères composites magnétisables de polymère vinylaromatique réticulé hydrophobe d'un diamètre de l'ordre de 50 à 10 000 nm et comprenant un coeur solide constitué de particules magnétisables et une écorce constituée d'un copolymère hydrophobe dérivé d'au moins un monomère vinylaromatique hydrophobe et d'au moins un polymère émulsifiant polyéthyléniquement insaturé soluble dans le ou les monomères vinylaromatiques et susceptible de réticuler avec le ou lesdits monomères. Toutefois, bien qu'elles puissent répondre à l'exigence de la taille, elles présentent l'inconvénient de ne pas avoir une répartition uniforme de la charge magnétique qui est localisée à l'intérieur du coeur. Par ailleurs, et comme cela ressort à l'évidence des figures annexées dans ce brevet, les particules ne sont pas homogènes en taille. Il s'agit donc d'un ensemble de particules polydisperses qui devront être fractionnées pour ne retenir que les particules de taille attendue. Enfin, du fait que les particules magnétisables à l'intérieur du coeur solide sont orientées de manière aléatoire et figées dans leur orientation, le moment magnétique résultant des microsphères composites correspond donc à la somme algébrique des moments des particules magnétisables avec, par voie de conséquence, une diminution du moment résultant lié à cette distribution aléatoire des particules à l'intérieur du coeur solide.

Comme on le verra ci-dessous, une des caractéristiques des nanosphères composites de l'invention, quand elles sont magnétiques, est que les nanoparticules dispersées à l'intérieur du coeur essentiellement liquide sont suffisamment mobiles pour que leur moment magnétique résultant facilite une séparation sous l'action d'un champ magnétique, même faible, ce qui présente un avantage incontestable par rapport aux particules magnétiques à coeur solide du type de celles décrites dans la demande de brevet EP.O 390 634. Ceci est particulièrement avantageux quand la teneur en nanoparticules magnétique est faible.

L'invention concerne donc de nouvelles nanosphères composites encapsulées qui pallient les inconvénients précités.

Les nanosphères composites encapsulées de l'invention contiennent au niveau de leur coeur une charge de matière inorganique répartie uniformément à l'intérieur de coeur, elles sont isodisperses en taille et sont susceptibles d'être utilisées dans des domaines aussi divers que la biologie, en particulier pour le diagnostic, la préparation de peintures, d'encres ou autres.

Les nanosphères composites de l'invention présentent un diamètre compris entre environ 50 et 1000 nm plus ou moins 5%, de préférence entre environ 100 et 500 nm plus ou moins 5% et avantageusement entre 100 et 200 nm plus ou moins 5% et comprennent :
- un coeur essentiellement liquide constitué d'une phase organique et de nanoparticules inorganiques distribuées de manière homogène à l'intérieur de la phase organique, et
- une enveloppe constituée au moins d'un polymère hydrophile qui est issu de la polymérisation d'au moins un monomère hydrosoluble, en particulier un N-alkylacrylamide, un N-N-dialkylacrylamide et plus particulièrement le N-isopropylacrylamide (NIPAM), le N-méthylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacrylamide.

Plus ou moins 5% signifie que le diamètre moyen en volume est défini à plus ou moins environ 5% près. La taille est mesurée par diffusion de la lumière.

Le coeur essentiellement liquide comprend :
- (i) un hydrocarbure aliphatique ou cyclique choisi parmi les composés comprenant de 5 à 12 atomes de carbone, leurs isomères et leurs mélanges. De préférence, l'hydrocarbure est choisi parmi le pentane, l'hexane, l'heptane, l'octane, le nonane, le décane, le undécane et le dodécane, étant entendu qu'il est à la portée de l'homme du métier d'adapter les conditions de polymérisation du procédé d'obtention en fonction du choix du ou des hydrocarbure(s) retenu(s). En particulier, quand la polymérisation est effectuée par une élévation de température, le montage réactionnel devra être adapté aux hydrocarbures volatils, tels que le pentane, et à la nature de l'amorceur de polymérisation choisi,
- (ii) des nanoparticules inorganiques choisies parmi les oxydes métalliques de fer, de titane, de cobalt, de zinc, de cuivre, de manganèse, de nickel ; la magnétite ; l'hématite, les ferrites telles que les ferrites de manganèse, nickel, manganèse-zinc; les alliages de cobalt, nickel ; les zéolites ; le talc ; les argiles telles que bentonite et kaolin ; l'alumine ; la silice ; le graphite ; le noir de carbone ou autres matériaux inorganiques. De préférence, les matériaux inorganiques sont choisis parmi les oxydes métalliques de fer, de titane, de cobalt, de zinc, de cuivre, de manganèse, de nickel ; la magnétite ; l'hématite, les ferrites telles que les ferrites de manganèse, nickel, manganèse-zinc; les alliages de cobalt, nickel.

Le coeur ainsi défini peut de plus comprendre un marqueur, tel qu'un marqueur fluorescent, luminescent ou radioactif, étant entendu que le marqueur est introduit lors de la préparation de l'émulsion telle que décrite dans l'exemple 1 qui suit.

Les nanoparticules inorganiques représentent de 5 à 95%, de préférence de 10 à 90%, de manière encore plus préférée de 20 à 80% et avantageusement de 50 à 80% en masse par rapport à la masse totale des nanosphères composites.

Dans un mode de réalisation de l'invention, l'enveloppe comprend un polymère hydrophile tel que défini ci-dessus qui constitue une couche externe de ladite enveloppe et un polymère hydrophobe qui constitue une couche interne de ladite enveloppe, située à l'interface entre la couche externe de l'enveloppe et le coeur essentiellement liquide.

Le polymère hydrophobe est choisi parmi les homopolymères de monomères vinylaromatiques insolubles dans l'eau, tels que styrène, méthylstyrène, éthylstyrène, tertio-butyl-styrène, vinyltoluène, ainsi que les copolymères de ces monomères entre eux et/ou avec d'autres comonomères, tels que les acrylates d'alkyle et les méthacrylates d'alkyle dans lequels le groupement alkyle comprend de 3 à 10 atomes de carbone, les esters d'acides éthyléniques possédant 4 ou 5 atomes de carbone et d'alkyle possédant 1 à 8 atomes de carbone, les acides méthacryliques, les dérivés styréniques, les composés diéniques.

Les nanosphères composites de l'invention trouvent notamment des applications dans les domaines de la peinture, des encres, des plastiques et, quand elles sont fonctionnalisées, dans des domaines divers de la biologie, en particulier pour la séparation de molécules biologiques ou biochimiques, pour des essais de diagnostic, pour la préparation de compositions thérapeutiques, prophylactiques ou cosmétiques.

Aussi, dans un mode de réalisation de l'invention les nanosphères composites présentent à la surface de l'enveloppe des groupements fonctionnels susceptibles d'interagir avec des molécules, par exemple des molécules biologiques, lesdits groupements fonctionnels étant apportés (i) soit par un traitement de la surface de l'enveloppe, par exemple chimique tel que par hydrolyse ou greffage de groupements fonctionnels, (ii) soit par addition d'au moins un monomère fonctionnel, tel que acide méthacrylique, acide acrylique, acide étaconique, aminoéthylméthacrylate, aminopropylméthacrylamide, (iii) soit par addition d'un amorceur fonctionnel, tel que le diméthyl 2,2'-azobis(2-méthylpropionate), le 4,4'-azobis(4-acide cyanovalérique) et le 2,2'-azobis(2-cyanopropanol).

Les nanosphères composites ainsi fonctionnalisées peuvent être utilisées pour la concentration d'acides nucléiques selon le protocole décrit dans la demande de brevet EP 0 842 184 ou pour la concentration de protéines conformément au protocole décrit dans la demande de brevet WO 99/35500.

Ainsi, les nanosphères composites de l'invention peuvent être fonctionnalisées et présenter à la surface de l'enveloppe des groupements fonctionnels réactifs, tels que des groupements carboxylique, amine, thiol, hydroxyl, tosyl, hydrazine, susceptibles de réagir avec au moins un ligand.

Les nanosphères composites fonctionnalisées ainsi formées seront susceptibles d'immobiliser un ligand, par exemple une molécule biologique, telle qu'un anticorps, un fragment d'anticorps, une protéine, un polypeptide, une enzyme, un polynucléotide, une sonde, une amorce, un fragment d'acide nucléique ; des molécules chimiques, telles que des polymères chimiques, des substances médicamenteuses, des molécules cages, des agents chélatants, des catalyseurs, la biotine.

La présente invention a également pour objet des conjugués dérivés des nanosphères composites de l'invention couplés à au moins un ligand tel que défini ci-dessus et leurs utilisations.

A titre d'exemple, lesdits conjugués sont utilisés dans des tests immunologiques pour la détection et/ou la quantification de protéines, d'antigènes, d'anticorps dans un échantillon biologique ou dans des essais utilisant la technologie des sondes pour la détection et/ou la quantification d'un acide nucléique ou d'un fragment d'acide nucléique dans un échantillon biologique. L'utilisation de sondes pour la détection et/ou la quantification d'un acide nucléique dans un échantillon est bien connue de l'homme du métier et on peut citer à titre d'illustration la technique d'hybridation sandwich. De même, les conjugués de l'invention peuvent être utilisés comme « agents porteurs d'amorces » pour une réaction d'amplification d'acides nucléiques dans un échantillon, par exemple par PCR (Polymerase Chain Reaction) ou toute autre technique d'amplification appropriée, permettant ainsi la détection et/ou la quantification d'acides nucléiques dans l'échantillon biologique.

La présente invention a donc également pour objet un réactif et une composition diagnostiques comprenant entre autre lesdites nanosphères composites ou lesdits conjugués et l'utilisation dudit réactif dans un essai analytique, par exemple pour la concentration de protéines ou d'acides nucléiques ou encore dans un essai diagnostique.

Les conjugués trouvent également une application dans le domaine thérapeutique ou prophylactique comme véhicule ou vecteur d'une substance médicamenteuse, d'un agent réparateur de gènes défectueux, d'un agent susceptible de bloquer ou d'inhiber l'expression d'un gène, tel qu'une sonde anti-sens en thérapie ou d'un agent susceptible de bloquer ou d'inhiber l'activité d'une protéine et de ce fait ils peuvent être utilisés dans une composition thérapeutique ou prophylactique.

Ainsi, les conjugués de l'invention sont susceptibles de véhiculer une substance médicamenteuse dans une composition thérapeutique ou prophylactique qui comprend ledit conjugué en association avec un adjuvant et/ou diluant et/ou excipient approprié et pharmaceutiquement acceptable, ladite substance médicamenteuse étant capable d'être relarguée *in vivo.* Les définitions des excipients et adjuvants pharmaceutiquement acceptables sont décrits par exemple dans Remington's Pharmaceutical Sciences 16^{th} ed., Mack Publishing Co.

Les conjugués de l'invention sont également susceptibles de véhiculer un gène d'intérêt thérapeutique codant pour au moins une protéine d'intérêt ou un fragment d'une protéine d'intérêt, étant entendu que par protéine on entend à la fois une protéine dans sa définition la plus généralement utilisée et un anticorps. Bien entendu, un tel conjugué est incorporé dans une composition thérapeutique ou prophylactique qui comprend également les éléments nécessaires à l'expression dudit gène d'intérêt thérapeutique.

Les conjugués de l'invention sont également utilisables, quand incorporés dans une composition thérapeutique ou prophylactique, pour le transfert *in vivo* de sondes ou oligonucléotides anti-sens. Les anti-sens sont capables d'interférer spécifiquement avec la synthèse d'une protéine cible d'intérêt, par inhibition de la formation et/ou du fonctionnement du polysome selon le positionnement de l'ARNm dans la cible. Donc le choix fréquent de la séquence entourant le codon d'initiation de la traduction comme cible pour une inhibition par un oligonucléotide anti-sens vise à prévenir la formation du complexe d'initiation. D'autres mécanismes dans l'inhibition par des oligonucléotides anti-sens impliquent une activation de la ribonucléase H qui digère les hybrides oligonucléotide anti-sens/ARNm ou une interférence au niveau de sites d'épissage par des oligonucléotides anti-sens dont la cible est un site d'épissage de l'ARNm. Les oligonucléotides anti-sens sont également complémentaires de séquences ADN et peuvent donc interférer au niveau de la transcription par la formation d'une triple hélice, l'oligonucléotide anti-sens s'appariant par des liaisons hydrogène dites de Hoogsteen au niveau du grand sillon de la double hélice d'ADN. Dans ce cas particulier, on parle plus précisément d'oligonucléotides antigènes. Il est bien entendu que les oligonucléotides anti-sens peuvent être strictement complémentaires de la cible ADN ou ARN à laquelle ils doivent s'hybrider, mais aussi non strictement complémentaires à la condition qu'ils s'hybrident à la cible. De même, il peut s'agir d'oligonucléotides anti-sens non modifiés ou modifiés au niveau des liaisons inter-nucléotidiques. Toutes ces notions font partie des connaissances générales de l'homme de l'art.

La présente invention concerne donc une composition thérapeutique comprenant, entre autres, un conjugué vecteur d'un oligonucléotide anti-sens tel que définis ci-dessus.

Enfin, les conjugués sont également susceptibles de former des complexes du type molécule cage/cryptate, chélatant/molécule chélatée ou de servir de véhicule pour des catalyseurs dans une application chimique.

Les nanosphères composites et les conjugués de l'invention sont obtenus par un procédé d'encapsulation par polymérisation d'une émulsion selon le protocole décrit dans les exemples qui suivent et l'invention concerne également un tel procédé de préparation.

Selon le procédé de l'invention, (i) on dispose d'une émulsion de départ stable et isodisperse constituée de deux phases non miscibles, une phase A hydrophobe constituée de gouttelettes contenant des nanoparticules inorganiques dispersées de manière homogène dans une phase organique contenant un agent tensio actif, ladite phase A étant dispersée dans une phase B hydrophile, (ii) on introduit dans la phase hydrophile B au moins un monomère hydrosoluble, un agent de réticulation hydrosoluble et un amorceur de polymérisation hydrosoluble, et (iii) on polymérise le monomère hydrosoluble en présence de l'agent de réticulation et de l'amorceur.

Dans un mode de réalisation de l'invention, préalablement à l'étape (ii) on introduit dans la phase hydrophile au moins un monomère hydrophobe et un premier amorceur de polymérisation hydrosoluble, puis on réalise l'addition dans la phase hydrophile d'au moins le monomère hydrosoluble et de l'agent de réticulation et éventuellement, si nécessaire, un deuxième amorceur de polymérisation hydrosoluble qui est identique ou différent du premier amorceur, étant entendu que l'addition d'un deuxième amorceur de polymérisation n'est utile que si la quantité du premier amorceur de polymérisation est limitée ou insuffisante pour conduire à bien la polymérisation complète. La quantité totale d'amorceur est comprise entre 1 et 10% molaire, de préférence entre 1 et 5% molaire par rapport à la concentration totale en monomères.

L'amorceur hydrosoluble est choisi parmi les sels de peroxydisulfate, c'est à dire les persulfates, tels que le persulfate de potassium, le persulfate de sodium et le persulfate d'ammonium ; les hydroperoxydes, tels que, l'hydroperoxyde de cumène ; le peroxyde d'hydrogène ; l'hydrochlorure de 2-2'-azobis-amidinopropane, le diméthyl 2,2'-azobis(2-méthylpropionate), le 4,4'-azobis(4-acide cyanovalérique) et le 2,2'-azobis(2-cyanopropanol). Parmi ceux ci, le diméthyl 2,2'-azobis(2-méthylpropionate), le 4,4'-azobis(4-acide cyanovalérique) et le 2,2'-azobis(2-cyanopropanol) sont des amorceurs fonctionnels. Les persulfates sont des amorceurs solubles dans l'eau. Une décomposition sous l'action de la chaleur génère des anions à radicaux sulfate qui contriburont à charger la nanosphère. Le peroxyde d'hydrogène se décompose dans l'eau pour former des radicaux hydroxyles qui ne sont pas chargés. Les hydroperoxydes sont solubles à la fois en phase aqueuse et dans des particules constituées de monomères. La décomposition des hydroperoxydes génère un hydroxyle et un autre radical oxygéné qui se partageront dans une des phases en fonction du type de peroxyde utilisé. L'hydroperoxyde de cumène dans le cas de la polymérisation de styrène est supposé se décomposer au niveau de l'interface entre la particule de monomères et l'eau, les radicaux hydroxyles entrent dans la phase aqueuse et les radicaux non polaires diffusent vers la particule. De la nature cationique ou anionique de l'amorceur dépendra le caractère cationique ou anionique de la nanosphère composite de l'invention et du conjugué résultant.

L'agent amorceur est introduit dans la phase hydrophile soit simultanément à l'introduction des monomères, soit préalablement à leur introduction, soit encore postérieurement à leur introduction.

Le monomère hydrosoluble et la monomère hydrophobe répondent aux définitions données précédemment.

L'agent de réticulation hydrosoluble est choisi parmi le N,N'-méthylènebisacrylamide (MBA) et l'éthylène glycol diméthacrylate.

La phase organique hydrophobe A est une phase comprenant un hydrocarbure aliphatique ou cyclique choisi parmi les composés comprenant de 5 à 12 atomes de carbone, leurs isomères et leurs mélanges. En particulier, l'hydrocarbure est choisi parmi le pentane, l'hexane, l'heptane, l'octane, le nonane, le décane, le undécane, le dodécane, étant entendu que lorsque la polymérisation est effectuée par élévation de la température, l'homme du métier doit adapter le montage réactionnel à des composants volatils, tels que le pentane, et à la nature de l'amorceur de polymérisation choisi. La phase B est une phase aqueuse, telle que de l'eau.

La polymérisation est effectuée de préférence par élévation de la température jusqu'à environ 60 à environ 90°C, de préférence à environ 70°C, en présence de l'amorceur de polymérisation, étant entendu que les conditions de polymérisation seront déterminées par l'homme du métier en fonction de la nature de l'amorceur choisi ; ou par photochimie à l'aide de rayonnements, tels que des rayonnements UV ou un faisceau laser ou d'autres sources d'énergie.

### Exemple 1 :

Une émulsion de départ stable et isodisperse a été préparée conformément à l'un ou l'autre des protocoles décrits dans cet exemple.
(i) L'émulsion primaire a été préparée à l'aide d'un procédé d'émulsification en incorporant progressivement, tout en cisaillant à l'aide d'un moulin colloïdal (Ika : nom commercial), la phase dispersée, formée de 45% en poids d'oxydes de fer dans de l'octane, à la phase continue formée de dodécyl sulfate de sodium à une concentration de 50% en poids dans l'eau jusqu'à l'obtention de fractions comprenant de 80% en poids de ferrofluide organique. Le mélange ainsi défini a été fragmenté dans une couette de type PG398 à un taux de cisaillement préalablement déterminé. L'émulsion primaire ainsi préparée est une émulsion polydisperse caractérisée par une distribution large du diamètre des gouttelettes qui est ensuite traitée par des tris magnétiques successifs pour l'obtention de l'émulsion de départ isodisperse en taille.
(ii) L'émulsion primaire a été préparée à l'aide d'un procédé d'émulsification en ajoutant rapidement la phase dispersée, formée d'octane, de 73% en poids d'oxydes de fer et d'un agent tensio-actif lipophile de type monoglycérol ou polyglycérol de polyrisinoléate (1 à 10% en poids), à la phase continue formée de tensio-actif de type tergitol NP10 (31 % en poids) grâce à une spatule. Le mélange ainsi défini est ensuite fragmenté dans une couette de type PG398 à un taux de cisaillement préalablement défini. L'émulsion primaire ainsi préparée est une émulsion relativement isodisperse caractérisée par une distribution faible du diamètre des gouttelettes qui est ensuite traitée par des tris magnétiques successifs pour l'obtention de l'émulsion de départ isodisperse en taille.

### Exemple 2 :

Dans un ballon de polymérisation de 25 ml, sont versés 20ml d'émulsion (1 % en poids dispersée dans du dodécylsulfate de sodium (SDS)à 0,8 fois la concentration micellaire critique (CMC) et dans l'eau). La solution est dégazée par bullage sous azote pour chasser l'air pendant 9 heures. 24 µl de monomères styrène et 4,3 mg de l'amorceur persulfate de potassium solubilisé dans 0,4 ml d'eau sont introduits et le mélange est gardé sous agitation pendant 2 heures. La température est ensuite élevée à 70°C, sous agitation pendant 20 minutes. Le mélange (280 mg de N-isopropylacrylamide solubilisé dans 1 ml d'eau, 11 mg de méthylène bisacrylamide solubilisé dans 0,4 ml d'eau, 30 µl d'acide méthacrylique) est introduit sur une période de 30 minutes. La polymérisation est conduite pendant 12 heures sous atmosphère d'azote et à une température de 70°C. La présence de groupements fonctionnels est assurée par l'acide méthacrylique.

Le latex magnétique final présente les caractéristiques suivantes à 20°C: Le diamètre déterminé par diffusion de lumière est de 192 nm plus ou moins 5 nm. Le taux d'oxyde de fer est d'environ 75%.

### Exemple 3 :

Dans un réacteur de polymérisation de 50 ml, sont versés 15 ml d'émulsion (0,7% dispersée dans du SDS à 1 fois la CMC et dans l'eau). La solution est dégazée par bullage sous azote pendant 3h:30 min. 7 µl de monomères styrène et 2 µl d'acide méthacrylique sont introduits et le mélange est gardé sous agitation pendant 20 minutes. L'amorceur (persulfate de potassium, 2 mg), solubilisé dans 0,1 ml d'eau est introduit et la solution est homogénéisée pendant 10 minutes. La température est ensuite élevée à 70°C, sous agitation pendant 25 minutes. Le mélange suivant (80 mg de N-isopropylacrylamide solubilisé dans 0,5 ml d'eau, 6 µl d'acide méthacrylique, 4 µl de styrène) est introduit comme suit :
Introduction de 200 µl de mélange et homogénéisation pendant 30 minutes suivie d'une introduction de 200 µl de mélange et homogénéisation pendant 30 minutes avant d'introduire les autres composants du mélange. La réaction de polymérisation est conduite sous agitation 300 tpm pendant 16 heures sous atmosphère d'azote et à une température de 70°C. La présence de groupements fonctionnels est assurée par l'acide méthacrylique.

Le latex magnétique final présente les caractéristiques suivantes: un diamètre de 187 nm à 20°C plus ou moins 5 nm déterminé par diffusion de lumière et un taux d'oxyde de fer d'environ 70%. Potentiel zeta -50 mV à pH 10 et 0 mv à pH 4,5.

### Exemple 4 :

Dans un ballon de polymérisation de 50 ml sont introduits 15 ml de d'une émulsion (0,7% dispersée dans l'eau à 1 fois la CMC en triton X405). Cette émulsion est préalablement dégazée par bullage sous azote pendant 5 heures. 7 µl de monomères styrène et de 2 mg de N-(3-aminopropyl)méthacrylamide solubilisé dans 0,2ml d'eau sont introduits. Le mélange est homogénéisé pendant 25 minutes avant d'introduire 2 mg de l'hydrochlorure de 2-2'-azobis-amidinopropane, l'amorceur, solubilisé dans 0,2 ml d'eau. Après homogénéisation de 20 minutes, la température est élevée à 70°C pendant 25 minutes et le mélange suivant (80 mg de N-isopropylacrylamide solubilisé dans 0,5 ml d'eau, 2 mg de méthylène bisacrylamide solubilisé dans 0,1 ml d'eau, 6 mg de N-(3-aminopropyl)méthacrylamide solubilisé dans 0.1 ml d'eau) est introduit comme de la façon suivante :
Introduction de 0,2 ml de mélange et homogénéisation pendant 20 minutes, introduction de 0,2 ml de mélange et homogénéisation pendant 30 minutes, introduction du reste du mélange.

La réaction de polymérisation est conduite sous agitation 300 tpm pendant 16 heures sous atmosphère d'azote et à une température de 70°C. La présence de groupements amines est assurée par le N-(3-aminopropyl)méthacrylamide.

Le latex magnétique final présente les caractéristiques suivantes: un diamètre de 187 nm plus ou moins 5 nm à 20°C déterminé par diffusion de lumière et un taux d'oxyde de fer de l'ordre de 70%. Potentiel zeta +50 mV à pH4, -50 mV à pH 10.

### Exemple 5 :

A 240 µl d'un latex magnétique à 3%, obtenu comme décrit précédemment, sont ajoutés successivement, 60 µl de Tween 20 (1 %), 636 µl de tampon phosphate (10 mM à pH 6,9), 60 µl de N-(3-diméthylaminopropyl)-N'-éthylcarbodiimidedihydrochlorure (25 mg/ml), 156 µl de N-hydroxysulfosuccinimide (25 mg/ml) et de la streptavidine (48 µl à 1 mg/ml).

Le mélange est incubé pendant une heure à température ambiante et les particules sont ensuite concentrées par application d'un champ magnétique, puis redispersées dans le tampon contenant du tensio-actif (phosphate 10 mM, pH 6,9 + Tween 20 0,05%).

9,9 µl d'un oligonucléotide biotinylé (ODN) de 17 mers présentant une masse de 5753 g/mole à une concentration de 338 nmoles/ml) sont ajoutés à 400 µl de particules recouvertes de streptavidine synthétisées précédemment pour constituer le témoin positif. 20 µl d'un oligonucléotide non biotinylé et non aminé de 17 mers (masse : 6452 g/mole à une concentration de 167 nmoles/ml) sont ajoutés à 400 µl de particules recouvertes de streptavidine synthétisées précédemment pour constituer le témoin négatif.

Les deux témoins sont incubés 30 minutes à température ambiante, séparés trois fois et redispersés la première fois avec un tampon basique (phosphate 10 mM, pH 9,9 + SDS, 5 fois la concentration micellaire critique, la seconde fois avec un tampon à pH neutre (phosphate 10 mM, pH 6,9 + Tween 20 0,05 %) et la troisième fois dans 280 µl de PEG contenant de l'ADN de sperme de saumon.

Dans les deux cas 20 µl d'ODN complémentaire à l'ODN du témoin positif marqués à la peroxydase de raifort (17 mers, concentration 9 nmoles/ml) sont ajoutés.

Les deux témoins sont à nouveau incubés une heure à température ambiante et sont encore séparés pour être dispersés de nouveau dans 400 µl de PEG contenant de l'ADN de sperme de saumon.

50 µl d'ortho-phénylènediamine sont ajoutés à 50 µl de particules. La réaction enzymatique est effectuée pendant 5 minutes et arrêtée par l'addition de 50 µl d'acide sulfurique (1M).

Les particules sont séparés du surnageant et ce dernier est dosé par méthode colorimétrique sur un appareil Axia Microreader (nom commercial, bioMérieux) à 492 et 630 nm.

Le témoin positif donne une densité optique de 2 000 unités de D.O, alors que le témoin négatif donne une densité de 1 000 unités de D.O.

La fluctuation de taille observée, avant et après polymérisation dans les exemples qui précèdent, est attribuée à la combinaison des deux phénomènes suivants : a) une éventuelle évaporation d'une parie de la phase organique et b) la conversion de la polymérisation d'un exemple à l'autre. Le taux d'oxyde de fer après polymérisation est sensiblement du même ordre de grandeur que dans l'émulsion utilisée avant polymérisation.

## Revendications

1. Nanosphères composites **caractérisées en ce qu'**elles présentent un diamètre compris entre environ 50 et 1000 nm plus ou moins 5 % et comprennent :
- un coeur essentiellement liquide constitué d'une phase organique et de nanoparticules inorganiques distribuées à l'intérieur de la phase organique, et
- une enveloppe constituée au moins d'un polymère hydrophile qui est issu de la polymérisation d'au moins un monomère hydrosoluble, en particulier un N-alkylacrylamide ou un N-N-dialkylacrylamide.

2. Nanosphères composites selon la revendication 1, **caractérisées en ce qu'**elles présentent un diamètre compris entre environ 100 et 500 nm plus ou moins 5%.

3. Nanosphères composites selon la revendication 1, **caractérisées en ce qu'**elles présentent un diamètre compris entre environ avantageusement entre 100 et 200 nm plus ou moins 5%.

4. Nanosphères composites selon l'une quelconque des revendications précédentes, **caractérisées en ce que** le monomère hydrosoluble est choisi parmi le N-isopropylacrylamide, le N-méthylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacrylamide.

5. Nanosphères composites selon l'une quelconque des revendications précédentes, **caractérisées en ce que** le coeur liquide comprend :
- (i) un hydrocarbure aliphatique ou cyclique choisi parmi les composés comprenant de 5 à 12 atomes de carbone, leurs isomères et leurs mélanges.
- (ii) des nanoparticules inorganiques choisies parmi les oxydes métalliques de fer, de titane, de cobalt, de zinc, de cuivre, de manganèse, de nickel ; la magnétite ; l'hématite, les ferrites telles que les ferrites de manganèse, nickel, manganèse-zinc; les alliages de cobalt, nickel ; les zéolites ; le talc ; les argiles telles que bentonite et kaolin ; l'alumine ; la silice ; le graphite ; le noir de carbone ou autres matériaux inorganiques.

6. Nanosphères composites selon la revendication 5, **caractérisées en ce que** les nanoparticules sont choisies parmi les oxydes métalliques de fer, de titane, de cobalt, de zinc, de cuivre, de manganèse, de nickel ; la magnétite ; l'hématite, les ferrites telles que les ferrites de manganèse, nickel, manganèse-zinc; les alliages de cobalt, nickel.

7. Nanosphères composites selon la revendication 5, **caractérisées en ce que** le coeur comprend de plus un marqueur, tels qu'un marqueur fluorescent, luminescent ou radioactif.

8. Nanosphères composites selon la revendication 5, **caractérisées en ce que** l'hydrocarbure est choisi parmi le pentane, l'hexane, l'heptane, l'octane, le nonane, le décane, le undécane et le dodécane.

9. Nanosphères composites selon l'une quelconque des revendications précédentes, **caractérisées en ce que** les nanoparticules inorganiques représentent de 5 à 95% en masse par rapport à la masse totale des nanosphères composites.

10. Nanosphères composites selon la revendication 9 **caractérisées en ce que** les nanoparticules inorganiques représentent de 10 à 90% en masse par rapport à la masse totale des nanosphères composites.

11. Nanosphères composites selon la revendication 9 **caractérisées en ce que** les nanoparticules inorganiques représentent de 20 à 80% en masse par rapport à la masse totale des nanosphères composites.

12. Nanosphères composites selon la revendication 9 **caractérisées en ce que** les nanoparticules inorganiques représentent de 50 à 80% en masse par rapport à la masse totale des nanosphères composites.

13. Nanosphères composites selon l'une quelconque des revendications précédentes, **caractérisées en ce que** l'enveloppe comprend un polymère hydrophile tel que défini dans les revendications 1 ou 4, ledit polymère constituant une couche externe de ladite enveloppe et un polymère hydrophobe qui constitue une couche interne de ladite enveloppe, située à l'interface entre la couche externe de l'enveloppe et le coeur essentiellement liquide.

14. Nanosphères composites selon la revendication 13, **caractérisées en ce que** le polymère hydrophobe est choisi parmi les homopolymères de monomères vinylaromatiques insolubles dans l'eau, tels que styrène, méthylstyrène, éthylstyrène, tertio-butyl-styrène, vinyltoluène, ainsi que les copolymères de ces monomères entre eux et/ou avec d'autres comonomères, tels que les acrylates d'alkyle et les méthacrylates d'alkyle dans lequels le groupement alkyle comprend de 3 à 10 atomes de carbone, les esters d'acides éthyléniques possédant 4 ou 5 atomes de carbone et d'alkyle possédant 1 à 8 atomes de carbone, les acides méthacryliques, les dérivés styréniques, les composés diéniques.

15. Nanosphères composites selon les revendications 1 à 14, **caractérisées en ce qu'**elles présentent de plus à la surface de l'enveloppe des groupements fonctionnels réactifs, tels que des groupements carboxylique, amine, thiol, aldéhyde, hydroxyl, tosyl, hydrazine, susceptibles de réagir avec au moins un ligand.

16. Conjugués dérivés des nanosphères composites selon la revendication 15, **caractérisées par le fait qu'**ils sont couplés à au moins un ligand choisi parmi un anticorps, un fragment d'anticorps, une protéine, un polypeptide, une enzyme, un polynucléotide, une sonde, une amorce, un fragment d'acide nucléique et la biotine.

17. Réactif, comprenant entre autre au moins une nanosphère telle que définie dans les revendications 1 à 15 ou un conjugué tel que défini dans la revendication 16.

18. Composition diagnostique comprenant entre autre un réactif tel que défini dans la revendication 17.

19. Utilisation d'un réactif tel que défini dans la revendication 17 ou d'une composition telle que définie dans la revendication 18 dans un essai de diagnostic.

20. Conjugués dérivés de nanosphères composites selon les revendications 1 à 14, **caractérisés en ce qu'**ils sont couplés à au moins un ligand choisi parmi les substances médicamenteuses, les sondes anti-sens, les agents réparateurs de gènes ou les gènes d'intérêt thérapeutique, les agents bloquant ou inhibant une activité protéique.

21. Composition thérapeutique ou prophylactique, **caractérisée en ce qu'**elle comprend, entre autre, un conjugué selon la revendication 20.

22. Utilisation d'un conjugué selon la revendication 20 pour la préparation d'une composition thérapeutique ou prophylactique.

23. Conjugués dérivés de nanosphères composites selon les revendications 1 à 13, **caractérisées par le fait qu'**elles sont couplées à au moins un ligand choisi parmi les molécules cages, les agents chélatants et les catalyseurs.

24. Procédé de préparation de nanosphères composites selon lequel,
(i) on dispose d'une émulsion de départ stable et isodisperse constituée de deux phases non miscibles, une phase A hydrophobe constituée de gouttelettes contenant des nanoparticules inorganiques dispersées de manière homogène dans une phase organique contenant un agent tensio actif, ladite phase A étant dispersée dans une phase B hydrophile,
(ii) on introduit dans la phase hydrophile B au moins un monomère hydrosoluble, un agent de réticulation hydrosoluble et un amorceur de polymérisation hydrosoluble, et on polymérise le monomère hydrosoluble en présence de l'agent de réticulation et de l'amorceur.

25. Procédé selon la revendication 24, selon lequel préalablement à l'étape (ii) on introduit dans la phase hydrophile au moins un monomère hydrophobe et un premier amorceur de polymérisation et on introduit, si nécessaire, à l'étape (ii) un deuxième amorceur de polymérisation.

26. Procédé selon les revendications 24 et 25, selon lequel le premier amorceur hydrosoluble de polymérisation et éventuellement le deuxième amorceur de polymérisation sont introduits dans la phase hydrophile soit simultanément à l'addition des monomères hydrosoluble et hydrophobe respectifs, soit préalablement ou postérieurement à l'addition des monomères hydrosoluble et hydrophobe respectifs.

27. Procédé selon l'une quelconque des revendications 24 à 26, dans lequel l'amorceur hydrosoluble est choisi parmi les sels de peroxydisulfate, c'est à dire les persulfates, tels que le persulfate de potassium, le persulfate de sodium et le persulfate d'ammonium ; les hydroperoxydes, tels que, l'hydroperoxyde de cumène; le peroxyde d'hydrogène ; l'hydrochlorure de 2-2'-azobis-amidinopropane, le diméthyl 2,2'-azobis(2-méthylpropionate), le 4,4'-azobis(4-acide cyanovalérique) et le 2,2'-azobis(2-cyanopropanol).

28. Procédé selon l'une quelconque des revendications 24 à 26, **caractérisé en ce que** le monomère hydrosoluble est choisi parmi les N-alkylacrylamide et N-N-dialkylacrylamide, en particulier le N-isopropylacrylamide, le N-méthylacrylamide, le N-éthylméthacrylamide, le N-n-propylacrylamide, le N-n-propylméthacrylamide, le N-isopropylméthacrylamide, le N-cyclopropylacrylamide, le N,N-diéthylacrylamide, le N-méthyl-N-isopropylacrylamide, le N-méthyl-N-n-propylacrylamide et le monomère hydrophobe est choisi parmi les monomères vinylaromatiques insolubles dans l'eau, tels que styrène, méthylstyrène, éthylstyrène, tertio-butyl-styrène, vinyltoluène, ainsi que les copolymères de ces monomères entre eux et/ou avec d'autres comonomères, tels que les acrylates d'alkyle et les méthacrylates d'alkyle dans lequels le groupement alkyle comprend de 3 à 10 atomes de carbone, les esters d'acides éthyléniques possédant 4 ou 5 atomes de carbone et d'alkyle possédant 1 à 8 atomes de carbone, les acides méthacryliques, les dérivés styréniques, les composés diéniques.

29. Procédé selon la revendication 24, **caractérisé en ce que** le monomère hydrophobe est choisi parmi les monomères vinylaromatiques insolubles dans l'eau, tels que styrène, méthylstyrène, éthylstyrène, tertio-butyl-styrène, vinyltoluène, ainsi que les copolymères de ces monomères entre eux et/ou avec d'autres comonomères, tels que les acrylates d'alkyle et les méthacrylates d'alkyle dans lequels le groupement alkyle comprend de 3 à 10 atomes de carbone, les esters d'acides éthyléniques possédant 4 ou 5 atomes de carbone et d'alkyle possédant 1 à 8 atomes de carbone, les acides méthacryliques, les dérivés styréniques, les composés diéniques.

30. Procédé selon l'une quelconque des revendications 24 à 29, dans lequel l'agent de réticulation hydrosoluble est choisi parmi le N,N'-méthylènebisacrylamide (MBA) et l'éthylène glycol diméthacrylate.

31. Procédé selon l'une quelconque des revendications 24 à 30, dans lequel la phase organique hydrophobe A est une phase comprenant un hydrocarbure aliphatique ou cyclique choisi parmi les composés comprenant de 5 à 12 atomes de carbone, leurs isomères et leurs mélanges, en particulier parmi le pentane, l'hexane, l'heptane, l'octane, le nonane, le décane, le undécane, le dodécane et la phase B est une phase aqueuse, en particulier de l'eau.

32. Procédé selon l'une des revendications 24 à 31, **caractérisée en ce que** la polymérisation est effectuée par élévation de la température jusqu'à environ 60 à environ 90°C, de préférence à environ 70 ° C, en présence de l'amorceur de polymérisation ou par photochimie à l'aide de rayonnements, tels que des rayonnements UV ou d'un faisceau laser.

## Patentansprüche

1. Verbundstoffnanosphären, **dadurch gekennzeichnet, dass** sie einen Durchmesser zwischen etwa 50 und 1.000 nm, ± 5 %, aufweisen und dass sie Folgendes aufweisen, nämlich
- einen im Wesentlichen flüssigen Kern, der aus einer organischen Phase und aus anorganischen Nanopartikeln gebildet ist, die in der organischen Phase verteilt sind, und
- eine Hülle, die aus zumindest einem hydrophilen Polymer gebildet ist, das aus der Polymerisation von zumindest einem wasserlöslichen Monomer insbesondere einem N-Alkylacrylamid oder einem N-N-Dialkylacrylamid entstanden ist.

2. Verbundstoffnanosphären nach Anspruch 1, **dadurch gekennzeichnet, dass** sie einen Durchmesser zwischen etwa 100 und 500 nm, ± 5 %, aufweisen.

3. Verbundstoffnanosphären nach Anspruch 1, **dadurch gekennzeichnet, dass** sie einen Durchmesser vorteilhafterweise zwischen etwa 100 und 200 nm, ± 5 %, aufweisen.

4. Verbundstoffnanosphären nach einem der vorhergehenden Ansprüche, **dadurch gekennzeichnet, dass** das wasserlösliche Monomer ausgewählt ist aus N-Isopropylacrylamid, N-Methylacrylamid, N-Ethylmethacrylamid, N-n-Propylacrylamid, N-n-Propylmethacrylamid, N-Isopropylmethacrylamid, N-Cyclopropylacrylamid, N,N-Diethylacrylamid, N-Methyl-N-isopropylacrylamid, N-Methyl-N-*n*-propylacrylamid.

5. Verbundstoffnanosphären nach einem der vorhergehenden Ansprüche, **dadurch gekennzeichnet, dass** der flüssige Kern Folgendes aufweist, nämlich
- (i) einen aliphatischen oder zyklischen Kohlenwasserstoff, ausgewählt aus den Verbindungen, die 5 bis 12 Kohlenstoffatome aufweisen, deren Isomeren und deren Mischungen
- (ii) anorganische Nanopartikel ausgewählt aus den metallischen Oxiden von Eisen, Titan, Kobalt, Zink, Kupfer, Mangan, Nickel; Magnetit; Hämatit; den Ferriten wie den Ferriten von Mangan, Nickel, Mangan-Zink; den Legierungen von Kobalt, Nickel; den Zeoliten; Talk; den Tonen wie Bentonit und Kaolin; Aluminiumoxid; Siliziumdioxid; Graphit; Ruß oder anderen anorganischen Materialien.

6. Verbundstoffnanosphären nach Anspruch 5, **dadurch gekennzeichnet, dass** die Nanopartikel ausgewählt sind aus den metallischen Oxiden von Eisen, Titan, Kobalt, Zink, Kupfer, Mangan, Nickel; Magnetit; Hämatit, den Ferriten wie den Ferriten von Mangan, Nickel, Mangan-Zink; den Legierungen von Kobalt, Nickel.

7. Verbundstoffnanosphären nach Anspruch 5, **dadurch gekennzeichnet, dass** der Kern zusätzlich noch einen Marker wie einen Fluoreszenz-, Lumineszenz- oder radioaktiven Marker aufweist.

8. Verbundstoffnanosphären nach Anspruch 5, **dadurch gekennzeichnet, dass** der Kohlenwasserstoff ausgewählt ist aus Pentan, Hexan, Heptan, Octan, Nonan, Decan, Undecan und Dodecan.

9. Verbundstoffnanosphären nach einem der vorhergehenden Ansprüche, **dadurch gekennzeichnet, dass** die anorganischen Nanopartikel bezogen auf das Gesamtgewicht der Verbundstoffnanosphären 5 bis 95 Gew.-% ausmachen.

10. Verbundstoffnanosphären nach Anspruch 9, **dadurch gekennzeichnet, dass** die anorganischen Nanopartikel bezogen auf das Gesamtgewicht der Verbundstoffnanosphären 10 bis 90 Gew.-% ausmachen.

11. Verbundstoffnanosphären nach Anspruch 9, **dadurch gekennzeichnet, dass** die anorganischen Nanopartikel bezogen auf das Gesamtgewicht der Verbundstoffnanosphären 20 bis 80 Gew.-% ausmachen.

12. Verbundstoffnanosphären nach Anspruch 9, **dadurch gekennzeichnet, dass** die anorganischen Nanopartikel bezogen auf das Gesamtgewicht der Verbundstoffnanosphären 50 bis 80 Gew.-% ausmachen.

13. Verbundstoffnanosphären nach einem der vorhergehenden Ansprüche, **dadurch gekennzeichnet, dass** die Hülle ein in den Ansprüchen 1 oder 4 definiertes hydrophiles Polymer aufweist, wobei dieses Polymer eine äußere Schicht der Hülle bildet und dass sie ein hydrophobes Polymer aufweist, das eine innere Schicht der Hülle bildet, die an der Grenzfläche zwischen der äußeren Schicht der Hülle und dem im Wesentlichen flüssigen Kern angeordnet ist.

14. Verbundstoffnanosphären nach Anspruch 13, **dadurch gekennzeichnet, dass** das hydrophobe Polymer ausgewählt ist aus den Homopolymeren der wasserunlöslichen vinylaromatischen Monomere, wie Styrol, Methylstyrol, Ethylstyrol, *tert*-Butylstyrol, Vinyltoluol, wie auch den Copolymeren dieser Monomere untereinander und/oder mit anderen Comonomeren, wie den Alkylacrylaten und den Alkylmethacrylaten, in denen die Alkylgruppe 3 bis 10 Kohlenstoffatome aufweist, den Estern von Ethylensäuren, die 4 oder 5 Kohlenstoffatome aufweisen und von Alkylen, die 1 bis 8 Kohlenstoffatome aufweisen, den Methacrylsäuren, den Styrolderivaten, den Dienverbindungen.

15. Verbundstoffnanosphären nach den Ansprüchen 1 bis 14, **dadurch gekennzeichnet, dass** sie zusätzlich an der Oberfläche der Hülle reaktive funktionelle Gruppen wie Carboxylgruppen, Amingruppen, Thiolgruppen, Aldehydgruppen, Hydroxylgruppen, Tosylgruppen, Hydrazingruppen aufweisen, die dazu neigen, mit zumindest einem Liganden zu reagieren.

16. Konjugate, die von Verbundstoffnanosphären nach Anspruch 15 abgeleitet sind, **dadurch gekennzeichnet, dass** sie mit zumindest einem Liganden gekoppelt sind, der ausgewählt ist aus einem Antikörper, einem Fragment eines Antikörpers, einem Protein, einem Polypeptid, einem Enzym, einem Polynukleotid, einer Sonde, einem Starter, einem Fragment einer Nukleinsäure, und Biotin.

17. Reagenz, das unter anderem zumindest eine in den Ansprüchen 1 bis 15 definierte Nanosphäre oder ein in Anspruch 16 definiertes Konjugat aufweist.

18. Diagnostische Zusammensetzung, die unter anderem ein im Anspruch 17 definiertes Reagenz aufweist.

19. Verwendung eines im Anspruch 17 definierten Reagenzes oder einer in Anspruch 18 definierten Zusammensetzung, in einem diagnostischen Assay.

20. Konjugate, die von Verbundstoffnanosphären nach den Ansprüchen 1 bis 14 abgeleitet sind, **dadurch gekennzeichnet, dass** sie mit zumindest einem Liganden gekoppelt sind, der ausgewählt ist aus den medikamentösen Substanzen, den Antisense-Sonden, den Genreparaturmitteln oder den Genen von therapeutischem Interesse, den Mitteln zum Blockieren oder Inhibitieren einer Proteinaktivität.

21. Therapeutische oder prophylaktische Zusammensetzung, **dadurch gekennzeichnet, dass** sie unter anderem ein Konjugat nach Anspruch 20 aufweist.

22. Verwendung eines Konjugats nach Anspruch 20 zur Herstellung einer therapeutischen oder prophylaktischen Zusammensetzung.

23. Konjugate, die von Verbundstoffnanosphären nach den Ansprüchen 1 bis 13 abgeleitet sind, **dadurch gekennzeichnet, dass** sie mit zumindest einem Liganden gekoppelt sind, der ausgewählt ist aus den Käfigmolekülen, den chelierenden Mitteln und den Katalysatoren.

24. Verfahren zum Herstellen von Verbundstoffnanosphären bei dem
(i) eine stabile und isodisperse Ausgangsemulsion bereitgestellt wird, die aus zwei nicht mischbaren Phasen gebildet wird, nämlich einer hydrophoben Phase A, die durch Tröpfchen gebildet wird, die anorganische Nanopartikel aufweisen, die auf homogene Weise in einer organischen Phase dispergiert sind, die einen grenzflächenaktiven Stoff aufweist, wobei die Phase A in einer hydrophilen Phase B dispergiert ist,
(ii) in die hydrophile Phase B zumindest ein wasserlösliches Monomer, ein wasserlösliches Vernetzungsmittel und ein wasserlöslicher Polymerisationsstarter eingebracht wird, und das wasserlösliche Monomer in Gegenwart des Vemetzungsmittels und des Starters polymerisiert wird.

25. Verfahren nach Anspruch 24, bei dem vor dem Schritt (ii) zumindest ein hydrophobes Monomer und ein erster Polymerisationsstarter in die hydrophile Phase eingebracht wird und, falls notwendig, in Schritt (ii) ein zweiter Polymerisationsstarter eingebracht wird.

26. Verfahren nach Anspruch 24 und 25, bei dem der erste wasserlösliche Polymerisationsstarter und gegebenenfalls der zweite Polymerisationsstarter entweder gleichzeitig mit der Zugabe der wasserlöslichen bzw. hydrophoben Monomere oder vor oder nach der Zugabe der wasserlöslichen bzw. hydrophoben Monomere, in die hydrophile Phase eingebracht wird.

27. Verfahren nach einem der Ansprüche 24 bis 26, bei dem der wasserlösliche Starter ausgewählt ist aus den Peroxydisulfatsalzen, nämlich den Persulfaten, wie Kaliumpersulfat, Natriumpersulfat und Ammoniumpersulfat; den Hydroperoxiden wie Cumolhydroperoxid; Wasserstoffperoxid; 2-2'-Azobisamidinpropanhydrochlorid, Dimethyl-2,2'-azobis(2-methylpropionat), 4,4'-Azobis(4-cyanvaleriansäure) und 2,2'-Azobis(2-cyanpropanol).

28. Verfahren nach einem der Ansprüche 24 bis 26, **dadurch gekennzeichnet, dass** das wasserlösliche Monomer ausgewählt ist aus den N-Alkylacrylamiden und den N-N-Dialkylacrylamiden, insbesondere N-Isopropylacrylamid, N-Methylacrylamid, N-Ethylmethacrylamid, N-*n* Propylacrylamid, N-n-Propylmethacrylamid, N-Isopropylmethacrylamid, N-Cyclopropylacrylamid, N,N-Diethylacrylamid, N-Methyl-N-isopropylacrylamid, N-Methyl-N-*n*-propylacrylamid und dass das hydrophobe Monomer ausgewählt ist aus den wasserunlöslichen vinylaromatischen Monomeren wie Styrol, Methylstyrol, Ethylstyrol, *tert*-Butylstyrol, Vinyltoluol, wie auch den Copolymeren dieser Monomere untereinander und/oder mit anderen Comonomeren wie den Alkylacrylaten und den Alkylmethacrylaten, in denen die Alkylgruppe 3 bis 10 Kohlenstoffatome aufweist, den Estern von Ethylensäuren, die 4 oder 5 Kohlenstoffatome aufweisen und von Alkylen, die 1 bis 8 Kohlenstoffatome aufweisen, den Methacrylsäuren, den Styrolderivaten und den Dienverbindungen.

29. Verfahren nach Anspruch 24, **dadurch gekennzeichnet, dass** das hydrophobe Monomer ausgewählt ist aus den wasserunlöslichen vinylaromatischen Monomeren wie Styrol, Methylstyrol, Ethylstyrol, *tert*-Butylstyrol, Vinyltoluol wie auch den Copolymeren dieser Monomere untereinander und/oder mit anderen Copolymeren wie den Alkylacrylaten und den Alkylmethacrylaten, in denen die Alkylgruppe 3 bis 10 Kohlenstoffatome aufweist, den Estern von Ethylensäuren, die 4 oder 5 Kohlenstoffatome aufweisen und von Alkylen, die 1 bis 8 Kohlenstoffatome aufweisen, den Methacrylsäuren, den Styrolderivaten, den Dienverbindungen.

30. Verfahren nach einem der Ansprüche 24 bis 29, bei dem das wasserlösliche Vernetzungsmittel ausgewählt ist aus N,N'-Methylenbisacrylamid (MBA) und Ethylenglycoldimethacrylat.

31. Verfahren nach einem der Ansprüche 24 bis 30, bei dem die hydrophobe organische Phase A eine Phase ist, die einen aliphatischen oder zyklischen Kohlenwasserstoff aufweist, der ausgewählt ist aus den Verbindungen, die 5 bis 12 Kohlenstoffatome aufweisen, deren Isomeren und deren Mischungen insbesondere aus Pentan, Hexan, Heptan, Octan, Nonan, Decan, Undecan, Dodecan und bei dem die Phase B eine wässrige Phase, insbesondere Wasser, ist.

32. Verfahren nach einem der Ansprüche 24 bis 31, **dadurch gekennzeichnet, dass** die Polymerisation durch Erhöhen der Temperatur auf etwa 60° bis etwa 90°C, vorzugsweise auf etwa 70°C in Gegenwart des Polymerisationsstarters oder durch Photochemie mit Hilfe von Strahlung wie UV-Strahlung oder einem Laserstrahl durchgeführt wird.

## Claims

1. Composite nanospheres, **characterized in that** they have a diameter of between about 50 and 1000 nm plus or minus 5%, and comprise:
- an essentially liquid core consisting of an organic phase and of inorganic nanoparticles distributed inside the organic phase, and
- an envelope consisting at least of one hydrophilic polymer which is derived from the polymerization of at least one water-soluble monomer, in particular an N-alkylacrylamide or an N,N-dialkylacrylamide.

2. Composite nanospheres according to Claim 1, **characterized in that** it has a diameter of between about 100 and 500 nm plus or minus 5%.

3. Composite nanospheres according to Claim 1, **characterized in that** it has a diameter advantageously of between about 100 and 200 nm plus or minus 5%.

4. Composite nanospheres according to any one of the preceding claims, **characterized in that** the water-soluble monomer is chosen from N-isopropylacrylamide, N-methylacrylamide, N-ethylmethacrylamide, N-n-propylacrylamide, N-n-propylmethacrylamide, N-isopropylmethacrylamide, N-cyclopropylacrylamide, N,N-diethylacrylamide, N-methyl-N-isopropylacrylamide, N-methyl-N-n-propylacrylamide.

5. Composite nanospheres according to any one of the preceding claims, **characterized in that** the liquid core comprises:
- (i) an aliphatic or cyclic hydrocarbon chosen from compounds comprising from 5 to 12 carbon atoms, isomers thereof and mixtures thereof.
- (ii) inorganic nanoparticles chosen from metal oxides of iron, titanium, cobalt, zinc, copper, manganese, nickel; magnetite; hematite, ferrites such as manganese, nickel and manganese-zinc ferrites; alloys of cobalt, nickel; zeolites; talc; clays such as bentonite and kaolin; alumina; silica; graphite; carbon black or other inorganic materials.

6. Composite nanospheres according to Claim 5, **characterized in that** the nanoparticles are chosen from metal oxides of iron, titanium, cobalt, zinc, copper, manganese, nickel; magnetite; hematite, ferrites such as the ferrites of manganese, nickel, manganese-zinc; alloys of cobalt, nickel.

7. Composite nanospheres according to Claim 5, **characterized in that** the core further comprises a marker, such as a fluorescent, luminescent or radioactive marker.

8. Composite nanospheres according to Claim 5, **characterized in that** the hydrocarbon is chosen from pentane, hexane, heptane, octane, nonane, decane, undecane and dodecane.

9. Composite nanospheres according to any one of the preceding claims, **characterized in that** the inorganic nanoparticles represent from 5 to 95% by mass relative to the total mass of the composite nanospheres.

10. Composite nanospheres according to Claim 9, **characterized in that** the inorganic nanoparticles represent from 10 to 90% by mass relative to the total mass of the composite nanospheres.

11. Composite nanospheres according to Claim 9, **characterized in that** the inorganic nanoparticles represent from 20 to 80% by mass relative to the total mass of the composite nanospheres.

12. Composite nanospheres according to Claim 9, **characterized in that** the inorganic nanoparticles represent from 50 to 80% by mass relative to the total mass of the composite nanospheres.

13. Composite nanospheres according to any one of the preceding claims, **characterized in that** the envelope comprises a hydrophilic polymer as defined in Claims 1 or 4, the said polymer constituting an external layer of the said envelope and a hydrophobic polymer which constitutes an internal layer of the said envelope, situated at the interface between the external layer of the envelope and the essentially liquid core.

14. Composite nanospheres according to Claim 13, **characterized in that** the hydrophobic polymer is chosen from homopolymers of vinylaromatic monomers which are insoluble in water, such as styrene, methylstyrene, ethylstyrene, tert-butylstyrene, vinyltoluene, as well as the copolymers of these monomers with each other and/or with other comonomers, such as alkyl acrylates and alkyl methacrylates in which the alkyl group comprises from 3 to 10 carbon atoms, the esters of ethylenic acids possessing 4 or 5 carbon atoms and alkyl possessing 1 to 8 carbon atoms, methacrylic acids, styrene derivatives, diene compounds.

15. Composite nanospheres according to Claims 1 to 14, **characterized in that** they further exhibit at the surface of the envelope reactive functional groups such as carboxyl, amino, thiol, aldehyde, hydroxyl, tosyl or hydrazine groups capable of reacting with at least one ligand.

16. Conjugates derived from the composite nanospheres according to Claim 15, **characterized in that** they are coupled to at least one ligand chosen from an antibody, an antibody fragment, a protein, a polypeptide, an enzyme, a polynucleotide, a probe, a primer, a nucleic acid fragment and biotin.

17. Reagent, comprising, inter alia, at least one nanosphere as defined in Claims 1 to 15 or a conjugate as defined in Claim 16.

18. Diagnostic composition comprising, inter alia, a reagent as defined in Claim 17.

19. Use of a reagent as defined in Claim 17 or of a composition as defined in Claim 18 in a diagnostic test.

20. Conjugates derived from the composite nanospheres according to Claims 1 to 14, **characterized in that** they are coupled to at least one ligand chosen from medicinal substances, antisense probes, gene repair agents or genes of therapeutic interest, agents blocking or inhibiting a protein activity.

21. Therapeutic or prophylactic composition, **characterized in that** it comprises, inter alia, a conjugate according to Claim 20.

22. Use of a conjugate according to Claim 20, for the preparation of a therapeutic or prophylactic composition.

23. Conjugate derived from the composite nanospheres according to Claims 1 to 13, **characterized in that** they are coupled to at least one ligand chosen from cage molecules, chelating agents and catalysts.

24. Method for preparing composite nanospheres according to which,
(i) a stable and isodisperse starting emulsion is available consisting of two imiscible phases, a hydrophobic phase A consisting of droplets containing inorganic nanoparticles homogeneously dispersed in an organic phase containing a surfactant, the said phase A being dispersed in a hydrophilic phase B,
(ii) at least one water-soluble monomer, one water-soluble crosslinking agent and one water-soluble polymerization initiator are introduced into the hydrophilic phase B, and the water-soluble monomer is polymerized in the presence of the crosslinking agent and the initiator.

25. Method according to Claim 24, according to which prior to step (ii), at least one hydrophobic monomer and a first polymerization initiator are introduced into the hydrophilic phase and if necessary, a second polymerization initiator is introduced at step (ii).

26. Method according to Claims 24 and 25, according to which the first water-soluble polymerization initiator and optionally the second polymerization initiator are introduced into the hydrophilic phase either simultaneously with the addition of the respective water-soluble and hydrophobic monomers, or prior to or subsequent to the addition of the respective water-soluble and hydrophobic monomers.

27. Method according to any one of Claims 24 to 26, in which the water-soluble initiator is chosen from peroxydisulphate salts, that is to say persulphates, such as potassium persulphate, sodium persulphate, ammonium persulphate; hydroperoxides, such as cumene hydroperoxide; hydrogen peroxide; 2,2'-azobisamidinopropane hydrochloride, dimethyl 2,2'-azobis(2-methylpropionate), 4,4'-azobis(4-cyanovaleric acid) and 2,2'-azobis(2-cyanopropanol).

28. Method according to any one of Claims 24 to 26, **characterized in that** the water-soluble monomer is chosen from N-alkylacrylamide and N-N-dialkylacrylamide, in particular N-isopropylacrylamide, N-methylacrylamide, N-ethylmethacrylamide, N-n-propylacrylamide, N-n-propylmethacrylamide, N-isopropylmethacrylamide, N-cyclopropylacrylamide, N,N-diethylacrylamide, N-methyl-N-isopropylacrylamide, N-methyl-N-n-propylacrylamide and the hydrophobic monomer is chosen from vinylaromatic monomers which are insoluble in water, such as styrene, methylstyrene, ethylstyrene tert-butylstyrene, vinyltoluene, as well as the copolymers of these monomers with each other and/or with other comonomers, such as alkyl acrylates and alkyl methacrylates in which the alkyl group comprises from 3 to 10 carbon atoms, the esters of ethylenic acids possessing 4 or 5 carbon atoms and of alkyl possessing 1 to 8 carbon atoms, methacrylic acids, styrene derivatives, diene compounds.

29. Method according to Claim 24, **characterized in that** the hydrophobic monomer is chosen from vinylaromatic monomers which are insoluble in water, such as styrene, methylstyrene, ethylstyrene tert-butylstyrene, vinyltoluene, as well as the copolymers of these monomers with each other and/or with other comonomers, such as alkyl acrylates and alkyl methacrylates in which the alkyl group comprises from 3 to 10 carbon atoms, the esters of ethylenic acids possessing 4 or 5 carbon atoms and of alkyl possessing 1 to 8 carbon atoms, methacrylic acids, styrene derivatives, diene compounds.

30. Method according to any one of Claims 24 to 29, in which the water-soluble crosslinking agent is chosen from N,N'-methylenebisacrylamide (MBA) and ethylene glycol dimethacrylate.

31. Method according to any one of Claims 24 to 30, in which the hydrophobic organic phase A is a phase comprising an aliphatic or cyclic hydrocarbon chosen from the compounds comprising from 5 to 12 carbon atoms, isomers thereof and mixtures thereof, in particular from pentane, hexane, heptane, octane, nonane, decane, undecane, dodecane and phase B is an aqueous phase, in particular water.

32. Method according to one of Claims 24 to 31, **characterized in that** the polymerization is carried out by raising the temperature up to about 60°C to about 90°C, preferably to about 70°C, in the presence of the polymerization initiator or by photochemistry using rays, such as UV rays or a laser beam.
